# EUROPÄISCHE PATENTSCHRIFT

(11) **EP 4 363 836 B1**
(45) Veröffentlichungstag und Bekanntmachung des Hinweises auf die Patenterteilung: **29.07.2026**
(21) Anmeldenummer: 22736262.1
(22) Anmeldetag: 28.06.2022
(51) Int. Cl.: G01N 27/04, H01J 37/30, H10P 30/22, G01T 1/26, G01T 1/28

(54) **VORRICHTUNG UND VERFAHREN ZUR BESTIMMUNG DES EINDRINGENS EINES TEILCHENS IN EIN MATERIAL**
DEVICE AND METHOD FOR DETERMINING THE PENETRATION OF A PARTICLE INTO A MATERIAL
DISPOSITIF ET PROCÉDURE POUR DÉTERMINER LA PÉNÉTRATION D'UNE PARTICULE DANS UN MATÉRIAU

(30) Priorität: 01.07.2021 DE 102021117027
(43) Veröffentlichungstag der Anmeldung: 08.05.2024
(73) Patentinhaber: SaxonQ GmbH, 04107 Leipzig (DE)
(72) Erfinder: ESQUINAZI, Pablo David, 04109 Leipzig (DE); MEIJER, Jan Berend, 04109 Leipzig (DE); KÜPPER, Johannes, 04109 Leipzig (DE)
(74) Vertreter: Hecht, Jan-David
(86) Internationale Anmeldenummer: PCT/EP2022/067789
(87) Internationale Veröffentlichungsnummer: WO 2023/275084

(56) Entgegenhaltungen:
- WO-A1-2021/011395
- CN-A- 106 680 328
- US-A- 5 451 529
- US-A1- 2007 252 240

## Beschreibung

Die vorliegende Erfindung betrifft eine Vorrichtung zur Bestimmung des Eindringens eines Teilchens in ein Material nach dem Oberbegriff von Anspruch 1 und ein Verfahren zur Bestimmung des Eindringens eines Teilchens in ein Material nach dem Oberbegriff von Anspruch 13.

Die deterministische Implantation von einzelnen Ionen ist eine der Schlüsseltechniken zur Herstellung von Quantensystemen und insbesondere von Quantencomputern in einem Festkörper. Durch immer kleiner werdende Bauelemente sind die deterministische Implantation von Ionen und ihre kontrollierte Platzierung auch für konventionelle Metalloxid-Feldeffekttransistoren (MOSFETs) von Interesse. Daneben werden auch optische Zentren, wie das Stickstoff-Vakanz-Zentrum in Diamant, für Quantencomputer oder als Einzelphotonenquelle erforscht. Auch dafür ist eine deterministische Implantation von Ionen von entscheidendem Vorteil für die vereinfachte technische Herstellung solcher Geräte.

Bisher bestehen zwei unterschiedliche Methodenklassen, um eine deterministische Implantation zu erreichen: Zum einen über eine Postdetektion und zum anderen über eine Predetektion.

Bei der sogenannten Postdetektion werden z.B. Sekundärelektronen, die beim Auftreffen eines Ions auf eine Oberfläche ausgelöst werden, detektiert. Das Vorhandensein solcher Sekundärelektronen ist daher ein Maß für die Implantation eines Ions. Alternativ können auch Elektronen-Loch-Paare mittels einer Diode abgesaugt und dadurch detektiert werden.

In beiden Fällen ist es aber von Nachteil, dass der Nachweis zu 100% gelingen muss, weil ansonsten Ionen doppelt platziert werden würden. Um messbare Effekte zu erzeugen, müssen allerdings hohe kinetische Energien der Ionen verwendet werden, wobei aufgrund der hohen kinetischen Energien die laterale Auflösung durch eine Deplatzierung mittels Straggling begrenzt ist. Außerdem ist die Anwendbarkeit dieser Methode auf bestimmte Substrate begrenzt. Schließlich erschwert das Straggling bei hohen kinetischen Energien die Nutzung des Gitterdefekts in späteren Anwendungen.

Eine solche Postdetektion ist beispielsweise in der EP 1 747 579 B1 und CN 109 786 198 A gezeigt.

Bei der sogenannten Predetektion wird das Ion bereits vor dem Auftreffen auf dem Target detektiert. Das Target ist dabei z.B. durch eine Gate-Spannung geschützt, so dass Ionen im Normalfall das Target nicht erreichen können. Nur wenn das Ion zuvor zweifelsfrei identifiziert wird, wird das Gate geöffnet.

In diesem Fall sind zwar keine "Falsch-Positiven" Signale möglich, allerdings "Falsch-Negative" Signale dadurch, wenn ein Ion zwar selektiert, aber nicht im Target Substrat ankommt, wodurch eine Fehlstelle ohne Fremdatom entsteht, die in einem zweiten Schritt nachgearbeitet werden müsste.

Eine solche Predetektion ist beispielsweise in der US 5 539 203 A und der CN 109 920 713 B gezeigt.

Diese Predetektion wird derzeit einerseits in lonenfallen genutzt, in der die einzelnen Ionen identifiziert und einzeln aus der Quelle geschossen werden. Andererseits erfolgt eine Nutzung auch in der Spiegelladungsmethode, bei der der Effekt Verwendung findet, dass geladene Teilchen Spiegelladungen erzeugen, die dann mittels hochempfindlicher Detektoren nachgewiesen werden. In beiden Fällen besteht aber die Notwendigkeit einer Detektion vor dem Target, so dass diese Methoden technisch sehr anspruchsvoll sind und deshalb bisher nur im Forschungsmaßstab umgesetzt wurden.

Alle bisher auf der Postdetektion oder der Predetektion basierenden Methoden sind außerdem nur sequentiell einsetzbar und daher nicht für einen hohen Durchsatz geeignet.

Die US 5 451 529 A offenbart ein Verfahren zur Echtzeitüberwachung von Ionenimplantationsdosen. Dabei wird der Anstieg des Widerstandes einer Metallsilizidschicht nach einer Ionenimplantation genutzt, um lonendosen im Bereich von 10¹³ bis 10¹⁶ Ionen cm⁻² zu kontrollieren.

Es ist daher Aufgabe der vorliegenden Erfindung, eine Vorrichtung und ein Verfahren bereitzustellen, mit denen das Eindringen von Teilchen in ein Material schnell und sicher detektiert werden kann. Insbesondere sollen mit dem Verfahren Teilchen deterministisch so in ein Substrat implantiert werden können, dass mit hoher örtlicher Auflösung und hohem Durchsatz gearbeitet werden kann. Vorzugsweise soll eine deterministische Teilchenquelle auf einfache und kostengünstige Art und Weise realisiert werden.

Diese Aufgabe wird gelöst mit der erfindungsgemäßen Vorrichtung nach Anspruch 1 und dem erfindungsgemäßen Verfahren nach Anspruch 13. Vorteilhafte Weiterbildungen sind in den abhängigen Ansprüchen und in der nachfolgenden Beschreibung zusammen mit den Figuren angegeben.

Erfinderseits wurde erkannt, dass diese Aufgabe in überraschender Art und Weise dadurch besonders einfach gelöst werden kann, wenn weder eine reine Postdetektion noch eine reine Predetektion durchgeführt werden, sondern beide Methoden miteinander kombiniert werden. Dies erfolgt dadurch, dass in Bezug auf die Strahlrichtung eines Teilchens, das in ein Material eindringen soll, zwischen Material und Teilchenquelle ein erste Schicht angeordnet wird, die nach Durchqueren des Teilchens ihren ohmschen Widerstand ändert, so dass anhand der Messung dieser Widerstandsänderung das Durchqueren des Teilchens und damit auch das Eindringen des Teilchens in das Material detektiert werden kann. Das Eindringen eines einzelnen Teilchens ist damit sicher feststellbar. Die erste Schicht kann direkt auf dem Material angeordnet sein, aber auch beabstandet vom Material bestehen. Die Beabstandung kann dabei durch ein oder mehrere Zwischenschichten bestehen, aber auch in Form einer lichten Beabstandung.

Es liegt somit keine reine Predetektion vor, weil zum Zeitpunkt der Messung des veränderten Widerstands das Teilchen schon in das Material eingedrungen ist. Es liegt aber auch keine reine Postdetektion vor, weil nicht die Wirkungen des Eindringens des Teilchens in das Material bestimmt werden. Somit liegt eine Kombination beider Methoden in neuer Form vor.

Die erfindungsgemäße Vorrichtung zur Bestimmung des Eindringens eines Teilchens aus einer Teilchenquelle in ein Material, ist dadurch gekennzeichnet, dass zwischen Teilchenquelle und Material eine erste Schicht angeordnet ist, die ihren Widerstand ändert, wenn das Teilchen die erste Schicht durchdringt, wobei Mittel zur Bestimmung der Widerstandsänderung der ersten Schicht bestehen. Die Bestimmung der Widerstandsänderung der ersten Schicht kann beispielsweise mittels Vierpunkt-Messung erfolgen.

In einer vorteilhaften Weiterbildung ist vorgesehen, dass sich die erste Schicht und das Material in ihrer chemischen Zusammensetzung unterscheiden. Dann ist das Eindringen besonders gut nachweisbar.

In einer vorteilhaften Weiterbildung ist vorgesehen, dass die erste Schicht und das Material beabstandet voneinander angeordnet sind. Auch dann ist der Nachweis besonders wirksam. Wenn zwischen Material und erster Schicht eine elektrisch isolierende Schicht angeordnet ist, ist der Nachweis selbst dann möglich, wenn das Material leitfähig ist.

In einer vorteilhaften Weiterbildung ist vorgesehen, dass die erste Schicht so ausgebildet ist, dass sich ihr ohmscher Widerstand nach Durchqueren des Teilchens dauerhaft, bevorzugt durch eine Zerstörung ihrer Gitterstruktur ändert. Wenn die erste Schicht beispielsweise aus Graphen oder Graphit besteht, dann unterscheidet sich die Querleitfähigkeit deutlich von der vertikalen Leitfähigkeit aufgrund der Gitterordnung dieser Schichtstruktur. Diese Gitterstruktur würde durch ein durchquerendes Teilchen dauerhaft zerstört werden, wodurch sich die Leitfähigkeit dauerhaft ändert. Dadurch ist der Nachweis langfristig möglich, was ein großer Vorteil gegenüber beispielsweise der Postdetektion nach EP 1 747 579 B1 und CN 109 786 198 A ist, da dort nur sehr kurzfristig Elektronen -Lochpaare erzeugt und wieder abgebaut werden. Dem entgegen kann durch die erfindungsgemäße Vorrichtung eine Detektion der Widerstandsänderung zu einem beliebig späteren Zeitpunkt stattfinden.

Ein weiterer Vorteil gegenüber dieser Art der Postdetektion besteht darin, dass keine Kühlung des Materials erforderlich ist, das Prinzip der vorliegenden Erfindung funktioniert auch bei Raumtemperatur. Außerdem können beliebige Materialen verwendet werden, weil darin keine Elektronen-Loch-Paare erzeugt werden müssen, sondern die Detektion über die erste Schicht erfolgt. Es muss sich also bei dem Material nicht zwingend um ein Halbleitermaterial handeln

In einer vorteilhaften Weiterbildung ist vorgesehen, dass die erste Schicht auf dem Material angeordnet ist. Dadurch kann das Eindringen besonders sicher festgestellt werden. Dabei kann die erste Schicht direkt aber auch unter Zwischenlage anderer Schichten, bspw. einer elektrisch isolierenden Schicht, auf dem Material aufliegen.

In einer vorteilhaften Weiterbildung ist vorgesehen, dass die erste Schicht als Gitterstruktur ausgebildet ist. Dadurch lassen sich viele Bereiche auf dem Material gleichzeitig auf ein Eindringen von Teilchen hin elektrisch überwachen, so dass eine Parallelisierung der Bestimmung des Eindringens ermöglicht wird.

In einer vorteilhaften Weiterbildung ist vorgesehen, dass die erste Schicht eine Schicht ist, deren elektrischer Widerstand sich in Bezug auf einen ungestörten Zustand gegenüber einem Zustand mit einem Atomgitterdefekt ändert, insbesondere eine Schicht ist, die Halbleitereigenschaften aufweist und bei Raumtemperatur eine kleine Energielücke (bevorzugt kleiner gleich 0,2 eV) und eine sehr geringe Leitungselektronendichte (bevorzugt kleiner gleich 10¹⁸ cm⁻³) besitzt. Dadurch kann das Eindringen auch für sehr kleine kinetische Teilchenenergien sicher festgestellt werden. Vorzugsweise ist die erste Schicht eine Schicht, die ihren elektrischen Widerstand nach Durchquerung eines Teilchens verringert. Alternativ könnte es aber auch eine Schicht sein, die ihren Widerstand erhöht.

Erfindungsgemäß ist vorgesehen, dass die erste Schicht einen der Stoffe umfasst aus der Gruppe: Graphit, Graphen, Bismut, Zinktellurid, Silberselenid und Quecksilbertellurid. Bei diesen Stoffen bewirken auch einzelne Atomgitterdefekte schon eine große und dauerhafte Änderung des ohmschen Widerstands, so dass auch Einzelteilchen mit geringer Energie leicht bestimmbar sind.

In einer vorteilhaften Weiterbildung ist vorgesehen, dass die erste Schicht eine Dicke im Bereich 5 nm bis 100 nm, bevorzugt im Bereich 15 nm bis 25 nm aufweist. Dann lassen sich Teilchen sehr gut detektieren ohne zugleich eine zu große Störung des Teilchens auf dem Weg zum Material zu bewirken, wodurch unter anderem eine exakte Positionierbarkeit des Teilchens sichergestellt ist.

In einer vorteilhaften Weiterbildung ist vorgesehen, dass die erste Schicht inselförmig ausgebildet ist, wobei die Inseln über elektrische Leitungen zur Widerstandsbestimmung verbunden sind, wobei die Inseln bevorzugt eine größere Dimension als die Leitungen aufweisen. Die Breite und Höhe der Leitungen liegt bevorzugt im Bereich von 5 nm bis 5 µm. Dann lässt sich eine Parallelisierung für einzelne Materialbereiche, die von den Inseln verdeckt bzw. in Bezug auf den Teilchenstrom verschattet sind, besonders gut umsetzen. Die Form der Inseln kann dabei rechteckig, rund, oval und dgl. mehr gestaltet sein. Die Leitungen und die Inseln können aus demselben Stoff bestehen (beispielsweise beide aus Graphit), müssen dies aber nicht (beispielsweise könnten die Inseln aus Graphit und die Leitungen aus einem Metall bestehen). In diesem Zusammenhang wird von "Stoff" gesprochen, um von dem "Material" zu unterscheiden, in das Teilchen implantiert werden. Tatsächlich handelt es sich bei "Stoff" aber um ein bestimmtes Material.

Die elektrischen Leitungen können gleichzeitig auch zur Steuerung eines Quantencomputers bzw. Quantensystems genutzt werden. Dazu könnte man beispielsweise die elektrischen Leitungen mit einer Wechselspannung beaufschlagen, um Mikrowellen zu erzeugen, die der Anregung bestimmter Energieniveau von NV-Zentren dienen.

In einer vorteilhaften Weiterbildung ist vorgesehen, dass die Inseln jeweils eine Dimension im Bereich 1 nm x 1 nm bis 500 nm x 500 nm, vorzugsweise eine Dimension im Bereich 10 nm x 10 nm bis 100 nm x 100 nm, bevorzugt eine Dimension im Bereich 15 nm x 15 nm bis 50 nm x 50 nm, bevorzugt eine Dimension im Bereich 20 nm x 20 nm bis 30 nm x 30 nm aufweisen. Dann lassen sich Teilchen in einem solchen Raster in das Material implantieren, dass damit Quantensysteme und letztlich Quantencomputer erzeugbar sind.

In einer vorteilhaften Weiterbildung ist vorgesehen, dass die Inseln einen Abstand voneinander im Bereich 10 nm bis 500 nm, bevorzugt im Bereich 20 nm bis 100 nm, insbesondere im Bereich 40 nm bis 60 nm aufweisen. Dann lassen sich Teilchen in einem solchen Raster in das Material implantieren, dass damit Quantensysteme und letztlich Quantencomputer erzeugbar sind.

In einer vorteilhaften Weiterbildung ist vorgesehen, dass zumindest eine Elektrode besteht. Dann können für elektrisch geladene Teilchen mit relativ geringer Energien ausreichend starke Gegenfelder erzeugt werden, so dass nach der Detektion eines in das Material an der Stelle der ersten Schicht eingedrungenen Teilchens weitere Teilchen am Eindringen in das Material durch das aufgebaute Gegenfeld gehindert werden. Dadurch ist eine deterministische Teilchenimplantation auf einfache und kostengünstige Art und Weise realisierbar, wenn die Elektrode in Abhängigkeit von der Detektion eines Teilchens geschaltet wird. Auf diese Weise können wirksame Gegenfelder für Teilchen mit Energien von kleiner gleich 100 keV erzeugt werden.

Diese Elektrode kann auf der zum Material gegenüberliegenden Seite der ersten Schicht, zwischen erster Schicht und Material oder auch auf der zur ersten Schicht gegenüberliegenden Seite des Materials angeordnet sein. Wenn die Elektrode zu dick und im Weg eines zu implantierenden Teilchens angeordnet sein sollte und daher als Maske für das zu implantierende Teilchen wirkt, sollte in der Elektrode zumindest eine Durchbrechung vorgesehen sein, um ein Durchqueren eines zu implantierenden Teilchens durch die Elektrode zu ermöglichen. Die Elektrode selbst kann auch als Maske eingesetzt werden.

Wenn mehrere Öffnungen zum Implantieren von Teilchen an unterschiedlichen Orten bestehen, dann müsste jeder Öffnung eine eigene Elektrode zugeordnet werden.

Für die Elektrode ist in üblicher Art und Weise eine Gegenelektrode vorzusehen, die beispielsweise an der Teilchenquelle angeordnet werden könnte.

In einer vorteilhaften Weiterbildung ist vorgesehen, dass die Teilchenquelle relativ zum Material bewegbar angeordnet ist. Auch dadurch kann eine deterministische Teilchenimplantation auf einfache und kostengünstige Art und Weise realisiert werden, wenn die Position der Teilchenquelle über dem Material in Abhängigkeit der Detektion eines Teilchens verändert wird, so dass an der Stelle eines schon implantierten Teilchens kein weiteres Teilchen mehr implantiert werden kann. Ein Vorteil bei Verwendung einer solchen beweglichen Teilchenquelle ist, dass nur die erste Schicht, d.h. die Detektionsschicht allein ausreicht. "Relativ beweglich" heißt in diesem Zusammenhang, dass eines der beiden Elemente Material und Teilchenquelle gegenüber dem anderen bewegt wird, während das andere Element entweder ortsfest ist oder ebenfalls bewegt werden kann.

In einer vorteilhaften Weiterbildung ist vorgesehen, dass die Teilchenquelle eine Apertur aufweist, die an einem AFM-Cantilever besteht. Dadurch kann die Teilchenquelle besonders ortsgenau Bereiche auf dem Material anfahren und dort Teilchen eindringen lassen. Eine solche Vorrichtung ist in der CN 109 920 713 B gezeigt, deren diesbezüglicher Inhalt vollumfänglich einbezogen wird. Im Gegensatz zur vorliegenden Erfindung beschreibt die CN 109 920 713 B allerdings eine Predetektion, bei der ein einzelnes Ion in einer Ionenfalle gefangen wird und erst dann die Implantation dieses Ions in einem Substrat erfolgt.

In einer vorteilhaften Weiterbildung ist vorgesehen, dass auf der zum Material gegenüberliegenden Seite der ersten Schicht zumindest eine Maske angeordnet ist, die für das Teilchen bei gewählten kinetischen Energien undurchdringbar ist, wobei die Maske bevorzugt die erste Schicht zumindest teilweise überdeckt, wobei die Maske insbesondere zumindest eine Öffnung aufweist, die einen Kanal zur Durchleitung des Teilchens durch die Maske definiert. Dadurch kann ortsgenau festgelegt werden, wo in dem Material das zu implantierende Teilchen implantiert wird. Das Maskenmaterial kann dabei auch die elektrischen Leitungen überdecken, die die erste Schicht zur Widerstandsmessung kontaktieren. Wenn eine Elektrode zur Erzeugung eines Gegenfeldes verwendet wird, kann die Elektrode zugleich die Maske bilden.

In einer vorteilhaften Weiterbildung ist vorgesehen, dass die Öffnung der Maske kleiner gleich 100 nm, vorzugsweise kleiner gleich 50 nm, bevorzugt kleiner gleich 10 nm, insbesondere kleiner gleich 5 nm ist. Dadurch ist die Ortsgenauigkeit sehr hoch und andererseits kann bei mehreren Implantationsbereichen das gewünschte Raster zwischen diesen implantierten Teilchen sehr genau reproduziert werden.

In einer vorteilhaften Weiterbildung ist vorgesehen, dass die Maske einen Thermoplast umfasst, bevorzugt PMMA. Dadurch kann die Maske besonders einfach und kostengünstig realisiert werden. Vor allem lassen sich sehr kleine, definierte Öffnungen darin mittels beispielsweise eines Lithographieverfahrens erzeugen.

In einer vorteilhaften Weiterbildung ist vorgesehen, dass zumindest eine durch Aufheizen schmelzbare zweite Schicht besteht, die für das Teilchen bei den gewählten kinetischen Energien undurchdringbar ist. Diese zweite Schicht kann mit der Maske identisch sein, muss dies aber nicht. Die zweite Schicht könnte also selbst die Maske bilden oder so angeordnet sein, dass ihr Schmelzen ein Verschließen einer Öffnung der Maske bewirkt. Die zweite Schicht muss daher keine zur Maske identische Öffnung aufweisen, kann dies allerdings. Wenn die zweite Schicht so in Bezug auf die Öffnung angeordnet ist, dass sie nach ihrem Aufschmelzen in die Öffnung eindringt und/oder die Öffnung überdeckt, muss die zweite Schichte keine identische Öffnung aufweisen, sondern kann beispielsweise einseitig neben der Öffnung in der Maske vorliegen. In der Regel wird man die Öffnung in der Maske beispielsweise durch lithografische Methoden sehr präzise herstellen können, während das in der zweiten Schicht beispielsweise durch Ätzen nicht so präzise möglich sein wird. Daher wird man die Öffnung in der zweiten Schicht eher größer dimensionieren als die Öffnungen in der Maske.

In einer vorteilhaften Weiterbildung ist vorgesehen, dass die zweite Schicht benachbart, vorzugsweise angrenzend an die Maske besteht, um so optimal einen Verschluss der Maskenöffnung zu bewirken, wobei die zweite Schicht bevorzugt zwischen Material und Maske angeordnet ist. Dann lässt sich eine Öffnung in der Maske besonders leicht mit dem geschmolzenen Stoff der zweiten Schicht überdecken bzw. verschließen (wenn das Material der zweiten Schicht in die Öffnung eindringt).

Auch dadurch kann eine deterministische Teilchenimplantation auf einfache und kostengünstige Art und Weise realisiert werden, wenn nach Bestimmung des Eindringens eines Teilchens sofort die zweite Schicht geschmolzen wird und dadurch eine Öffnung der Maske verschlossen wird, so dass an der Stelle eines schon implantierten Teilchens kein weiteres Teilchen mehr implantiert werden kann. Diese Schmelzschicht kann ggf. auch die elektrischen Leitungen der ersten Schicht überdecken.

In einer vorteilhaften Weiterbildung ist vorgesehen, dass ein Heizelement zum Aufheizen der zweiten Schicht besteht. Dieses Heizelement kann beispielsweise als gesonderte Schicht bestehen, es könnte allerdings auch durch die erste Schicht gebildet werden, wenn diese ein ohmscher Heizer, wie Graphit ist. Dann könnten die Leitungen zur Widerstandsmessung für die Speisung des Heizelements verwendet werden. Dadurch kann die zweite Schicht besonders einfach geschmolzen werden. Wenn das Heizelement durch die erste Schicht gebildet wird, ist die Vorrichtung besonders kompakt aufgebaut. Es könnte anstelle eines eigenen Heizelements der Vorrichtung aber auch ein Aufschmelzen der zweiten Schicht mit Hilfe eines LASER-Strahls oder eines Elektronenstrahls erfolgen.

In einer vorteilhaften Weiterbildung ist vorgesehen, dass die zweite Schicht einen Schmelzpunkt von kleiner gleich 100 °C aufweist. Dadurch kann das Aufschmelzen der zweiten Schicht und damit das Verhindern eines weiteren Eindringens von Teilchen besonders schnell bewirkt werden, so dass die Vorrichtung nur sehr geringe Ansprechzeiten aufweist.

In einer vorteilhaften Weiterbildung ist vorgesehen, dass die zweite Schicht einen der Stoffe umfasst aus der Gruppe: Wachs und Polymer, wobei das Polymer bevorzugt Polycaprolactone (PCL) ist. Dadurch kann die zweite Schicht besonders einfach und kostengünstig realisiert werden.

In einer vorteilhaften Weiterbildung ist vorgesehen, dass das Material einen der Stoffe umfasst aus der Gruppe: Diamant, SiC, Silizium, TiO₂ und ZnO. Dann können besonders leicht Quantensysteme erzeugt werden.

Unabhängiger Schutz wird beansprucht für das erfindungsgemäße Verfahren zur Bestimmung des Eindringens eines Teilchens aus einer Teilchenquelle in ein Material, das dadurch gekennzeichnet ist, dass zwischen Teilchenquelle und Material eine erste Schicht angeordnet wird, die ihren Widerstand ändert, wenn das Teilchen die erste Schicht durchdringt, wobei die Widerstandsänderung der ersten Schicht bestimmt wird. Wird dann eine Widerstandsänderung der ersten Schicht, beispielsweise im Rahmen einer Vierpunktmessung festgestellt, dann kann auf die Implantation eines solchen Teilchens geschlossen werden.

In einer vorteilhaften Weiterbildung ist vorgesehen, dass die erfindungsgemäße Vorrichtung verwendet wird. Dadurch ist das erfindungsgemäße Verfahren besonders einfach umsetzbar.

In einer vorteilhaften Weiterbildung ist vorgesehen, dass das Material in Vakuum gehalten wird, wobei der maximale Druck bevorzugt kleiner oder gleich 0,0001 mbar ist. Dann wird die Ortspräzision nochmals gesteigert, weil das Teilchen keinen unnötigen Stößen mit Teilchen einer Atmosphäre unterworfen wird, wodurch sowohl die seitliche Positionspräzision als auch die Tiefenpräzision leiden können.

In einer vorteilhaften Weiterbildung ist vorgesehen, dass das Material einen der Stoffe umfasst aus der Gruppe: Diamant, SiC, Silizium, TiO₂ und ZnO. Dann können besonders leicht Quantensysteme erzeugt werden.

In einer vorteilhaften Weiterbildung ist vorgesehen, dass das Verfahren zur deterministischen Ionenimplantation, bevorzugt zur Erzeugung von Quantensystemen, verwendet wird. Dazu könnte beispielsweise wiederum die zweite Schicht eingesetzt werden, um eine weitere Ioneneinstrahlung zu verhindern. Dieses Verhindern könnte aber auch durch eine Elektrode erfolgen, die beispielsweise als Maske vorliegt oder auch an der Teilchenquelle, so dass das Anlagen einer Spannung daran das Eindringen weiterer Teilchen in das Material verhindert. Hierzu könnte auch eine deterministische Teilchenquelle verwendet werden, deren Teilchenausstoß gezielt geregelt werden kann.

In einer vorteilhaften Weiterbildung ist vorgesehen, dass das Verfahren zur ortsaufgelösten Einzelteilchendetektion verwendet wird.

In einer vorteilhaften Weiterbildung ist vorgesehen, dass das Verfahren für die ortsaufgelöste Massenspektroskopie verwendet wird. Dabei werden einzelne Ionen durch die Detektion der Widerstandsänderung bestimmt.

In einer vorteilhaften Weiterbildung ist vorgesehen, dass das Verfahren für die Erzeugung magnetischer Momente im Material und bevorzugt ihre Detektion durch die Messung des lokalen Magnetowiderstands der ersten Schicht verwendet wird. Die dazu notwendige deterministische Implantation von Defekten bzw. Ionen mit magnetischen Momenten würde durch das erfindungsgemäße Verfahren erfolgen. In Abhängigkeit von der Fläche der Inseln der ersten Schicht wäre die Detektion eines magnetischen Moments von einem µ_{B} (ein Bohr Magneton) möglich. Genauer gesagt kann eine Änderung des Widerstandes durch ein Magnetfeld, also der Magnetowiderstand, direkt nach der Implantation gemessen werden, wenn der Defekt bzw. das implantierte Ion ein magnetisches Moment hat. Dieses magnetische Moment erzeugt ein Streufeld auf der Oberfläche der Probe und dieses beeinflusst den Widerstand der z.B. ersten Schicht (beispielsweise einer Graphitschicht). Je kleiner die Fläche der ersten Schicht desto besser die Empfindlichkeit zum Streufeld, wenn zugleich auch die Kontakte enger liegen. Diese Änderung ist zeitunabhängig, da das Streufeld immer da ist.

In einer vorteilhaften Weiterbildung ist vorgesehen, dass das Verfahren für die ionenstrahlgestützte Materialforschung verwendet wird. Dabei geht es um die Möglichkeit, einzelne Ionen verschiedener Massen auf verschiedenen Materialien zu implantieren. Mit dem erfindungsgemäßen Verfahren wird dann es möglich sein, die erzeugten Effekte auf das implantierte Material besser zu untersuchen, z.B. Eindringtiefe vs. Ionenenergie bis zu erzeugter Unordnung (Defekte) im Materialgitter. Man kann somit deterministisch diese einzelnen Ionen implantieren und den Effekt dieser Implantation auf das Material mit anderen Untersuchungsmethoden untersuchen.

Ebenfalls kann das erfindungsgemäße Verfahren zur Alpha-Teilchen-Detektion genutzt werden.

Weiterhin wird unabhängiger Schutz beansprucht für die erfindungsgemäße Verwendung der erfindungsgemäßen Vorrichtung zur Herstellung eines Detektors für die Massenspektroskopie, eines Detektors für die Einzelteilchenimplantation, eines Informationsspeichers oder eines Bauteils für einen Quantencomputer, bevorzugt einen Prozessor für einen Quantencomputer.

Zugleich wird auch selbstständiger Schutz beansprucht für einen Detektor für die Massenspektroskopie, einen Detektor für die Einzelteilchenimplantation, einen Informationsspeicher und ein Bauteil für einen Quantencomputer, bevorzugt einen Prozessor für einen Quantencomputer, die die erfindungsgemäße Vorrichtung aufweisen.

Insgesamt ist somit festzustellen, dass die vorliegende Erfindung für zahlreiche Anwendungsfälle verwendet werden kann:
Zum einen kann eine reine Teilchendetektion erfolgen. Hierzu ist nur die erste Schicht notwendig.

Zum anderen kann eine deterministische Teilchenimplantation dadurch erfolgen, dass der Ort, an dem ein Teilchen implantiert wird, für nachfolgende Teilchen gesperrt wird.

Dieses Sperren kann durch eine elektrostatische Sperre oder eine Materialsperre erfolgen.

Im Rahmen der Materialsperre ist das Aufschmelzen einer zweiten Schicht möglich, was durch ohmsche Heizung, aber auch durch Energieeintrag über einen LASER-Strahl oder einen Elektronenstrahl erfolgen könnte. Die Materialsperre kann aber auch durch Aufwachsen eines bestimmten Materials, beispielsweise aus einem Precursur-Gas im Zusammenspiel mit einem Elektronenstrahl oder einem Ionenstrahl, erfolgen.

Schließlich kann die deterministische Teilchenimplantation auch durch eine bewegliche lonenstrahlquelle erfolgen, die nach Feststellung eines wunschgemäß implantierten Teilchens abgeschaltet bzw. wegbewegt wird.

Diese Vorgehensweisen können auch miteinander kombiniert werden.

Weiterhin können mit der erfindungsgemäßen Vorrichtung auch Detektoren für die Massenspektroskopie, Detektoren für die Einzelteilchenimplantation, Informationsspeicher allgemein und Bauteile für einen Quantencomputer, bevorzugt Prozessoren für einen Quantencomputer hergestellt werden.

Die Merkmale und weitere Vorteile der vorliegenden Erfindung werden im Folgenden anhand der Beschreibung zweier bevorzugter Ausführungsbeispiele im Zusammenhang mit den Figuren deutlich werden. Dabei zeigen rein schematisch:
- Fig. 1: den erfindungsgemäßen Sensor nach einer ersten bevorzugten Ausgestaltung in einer ausschnittsweisen Querschnittsansicht,
- Fig. 2: den erfindungsgemäßen Sensor nach Fig. 1 in einer ausschnittsweisen Draufsicht,
- Fig. 3: den erfindungsgemäßen Sensor nach Fig. 1 in einer Draufsicht,
- Fig. 4: den erfindungsgemäßen Sensor nach Fig. 1 in einem ersten Betriebszustand,
- Fig. 5: den erfindungsgemäßen Sensor nach Fig. 1 in einem zweiten Betriebszustand,
- Fig. 6: den erfindungsgemäßen Sensor nach Fig. 1 in einem dritten Betriebszustand,
- Fig. 7: den erfindungsgemäßen Sensor nach Fig. 1 in einem vierten Betriebszustand,
- Fig. 8: den erfindungsgemäßen Sensor nach einer zweiten bevorzugten Ausgestaltung in einer ausschnittsweisen Querschnittsansicht,
- Fig. 9: den erfindungsgemäßen Sensor nach Fig. 8 in einem ersten Betriebszustand,
- Fig. 10: den erfindungsgemäßen Sensor nach Fig. 8 in einem zweiten Betriebszustand und
- Fig. 11: den erfindungsgemäßen Sensor nach einer dritten bevorzugten Ausgestaltung in einer ausschnittsweisen Querschnittsansicht.

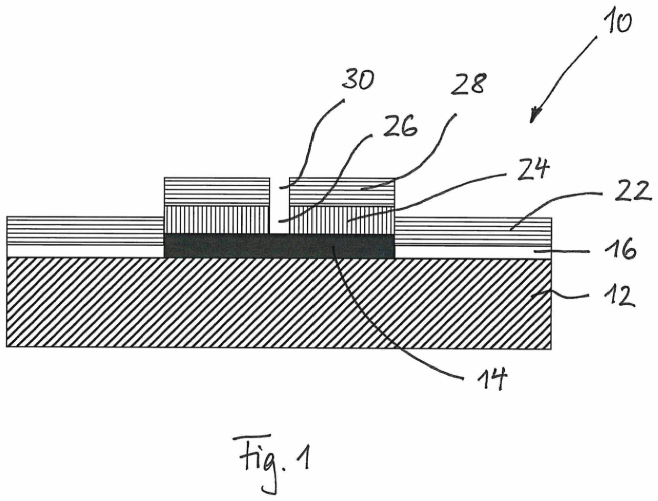

In den Fig. 1, 2 und 3 ist die erfindungsgemäße Vorrichtung 10 als Sensor 10 gemäß einer ersten bevorzugten Ausgestaltung in verschiedenen Ansichten gezeigt.

Es ist zu erkennen, dass der Sensor 10 ein Substrat 12 aufweist, auf dem eine erste Schicht inselförmig 14, 14a, 14b, 14c, ..., 14n angeordnet ist. Diese Inseln 14, 14a, 14b, 14c, ..., 14n sind rasterförmig angeordnet und jeweils mit elektrischen Leitungen 16, 16a, 16b, 16c, ..., 16n, 18, 18a, 18b, 18c, ..., 18n kontaktiert, so dass sich eine Gitterstruktur 20 ergibt.

Die Leitungen 16, 16a, 16b, 16c, ..., 16n, 18, 18a, 18b, 18c, ..., 18n sind von einem Maskenmaterial 22 überdeckt.

Auf den Inseln 14, 14a, 14b, 14c, ..., 14n befindet sich eine zweite Schicht 24, die jeweils über jeder Insel 14, 14a, 14b, 14c, ..., 14n eine zentrale Öffnung 26 aufweist.

Auf der zweiten Schicht 24 befindet sich eine Maske 28, die identisch zur zentralen Öffnung 26 der zweiten Schicht 24 jeweils eine zentrale Öffnung 30 aufweist. Die jeweiligen zentralen Öffnungen 26, 30 von zweiter Schicht 26 und Maske 28 sind somit über der entsprechenden Insel 14, 14a, 14b, 14c, ..., 14n in Linie angeordnet.

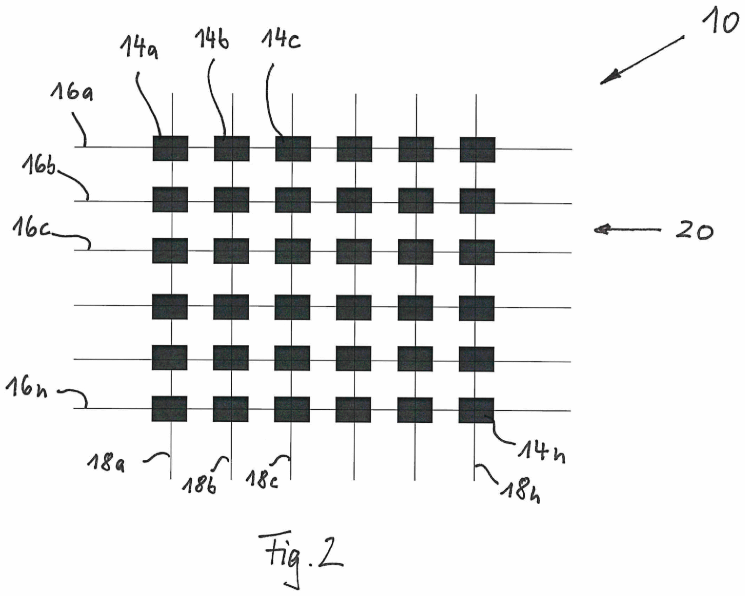

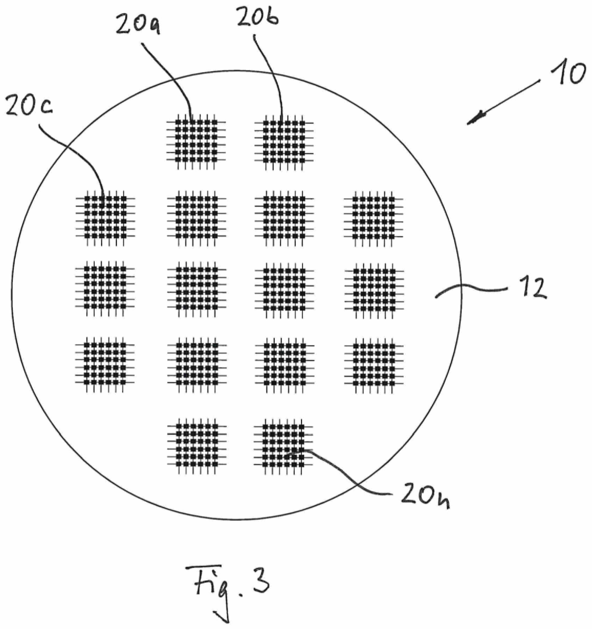

In den Fig. 2 und 3 sind nur die auf dem Substrat 12 angeordneten Gitterstrukturen 20, 20a, 20b, 20c, ..., 20n mit der ersten Schicht 14, 14a, 14b, 14c, ..., 14n und den elektrischen Leitungen 16, 16a, 16b, 16c, ..., 16n, 18, 18a, 18b, 18c, ..., 18n gezeigt, nicht jedoch das Maskenmaterial 22, die zweite Schicht 24 und die Maske 28.

Der Sensor 10 kann - wie in Fig. 3 gezeigt - mit zahlreichen Gitterstrukturen 20a, 20b, ... 20n bestehen, allerdings könnten die Gitterstrukturen 20a, 20b, ... 20n auch miteinander direkt verbunden sein, so dass sich die in Fig. 2 gezeigte Gitterstruktur 20 über das gesamte Substrat 12 in Fig. 3 aufspannt.

Das Substrat 12 kann beispielsweise eine Diamantschicht sein. Bei der ersten Schicht 14 handelt es sich vorzugsweise um eine Graphitschicht mit einer Dicke von 5 nm bis 100 nm, bevorzugt von 15 nm bis 25 nm.

Die Inseln 14, 14a, 14b, 14c, ..., 14n weisen eine quadratische Form mit einer Kantenlänge im Bereich 1 nm bis 500 nm, bevorzugt im Bereich 20 nm bis 30 nm auf. Die Inseln 14, 14a, 14b, 14c, ..., 14n sind dabei voneinander 10 nm bis 500 nm, bevorzugt 40 nm bis 60 nm beabstandet. Die Form der Inseln 14, 14a, 14b, 14c, ..., 14n kann allerdings auch rund, oval und dgl. mehr gestaltet sein.

Die elektrischen Leitungen 16, 16a, 16b, 16c, ..., 16n, 18, 18a, 18b, 18c, ..., 18n können metallisch sein, sind im gezeigten Ausführungsbeispiel allerdings ebenfalls aus Graphit, so dass sie in einem Arbeitsgang mit den Inseln 14, 14a, 14b, 14c, ..., 14n hergestellt werden können. Die elektrischen Leitungen 16, 16a, 16b, 16c, ..., 16n, 18, 18a, 18b, 18c, ..., 18n weisen somit eine Länge im Bereich 10 nm bis 500 nm, bevorzugt im Bereich 40 nm bis 60 nm auf. Ihre Breite und Höhe liegt im Bereich von 5 nm bis 100 nm, bevorzugt von 15 nm bis 25 nm.

Alternativ dazu, dass die elektrischen Leitungen 16, 16a, 16b, 16c, ..., 16n, 18, 18a, 18b, 18c, ..., 18n in einer Ebene an die Inseln 14, 14a, 14b, 14c, ..., 14n anschließen könnten sie auch unter- und/oder oberhalb der Inseln 14, 14a, 14b, 14c, ..., 14n angeordnet sein.

Als Maskenmaterial 22 und als Material für die Maske 28 wird PMMA mit einer Dicke im Bereich von 5 nm bis 5µm verwendet.

Die zweite Schicht 24 besteht aus einem Polymer und weist eine Dicke im Bereich von 1 nm bis 5 µm auf. Der Schmelzpunkt liegt bei 100 °C.

Die Öffnungen 26, 30 besitzen einen kreisrunden Querschnitt mit einem Durchmesser kleiner 100 nm, bevorzugt kleiner 50 nm, vorzugsweise kleiner 10 nm, insbesondere kleiner 5 nm. Der Querschnitt kann allerdings auch eckig, oval und dgl. mehr gestaltet sein.

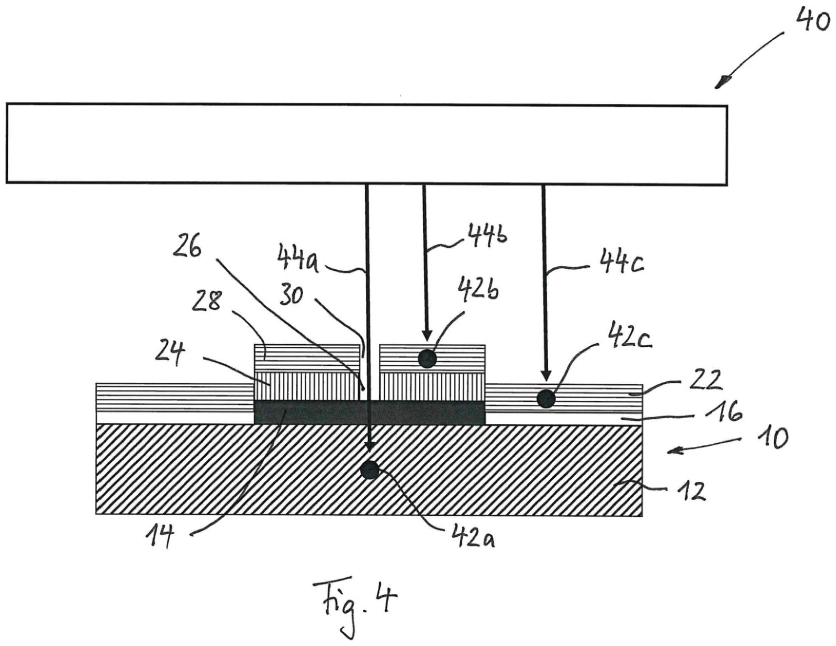

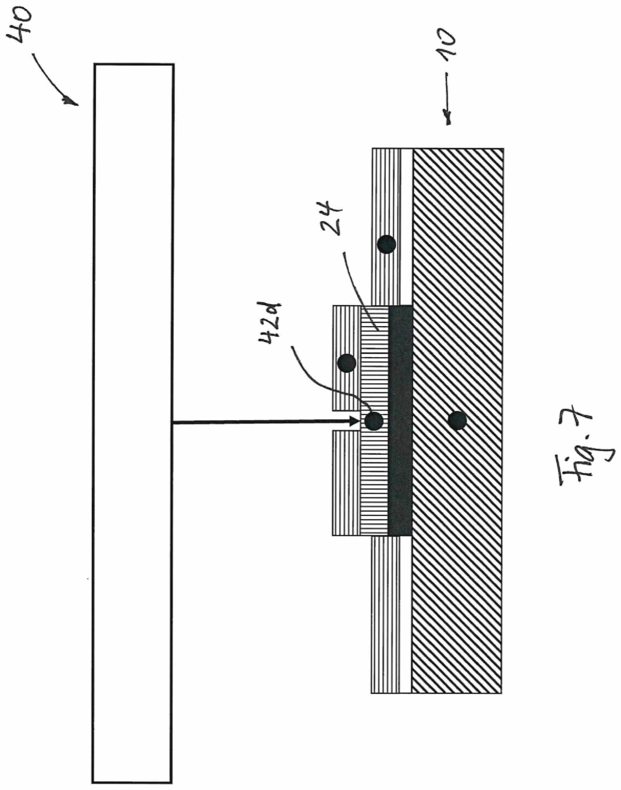

Im Zusammenhang mit den Fig. 4 bis 7 wird nun die Funktionsweise bzw. die Verwendung des Sensors 10 beschrieben.

In Fig. 4 ist zu erkennen, dass der Sensor 10 in Verbindung mit einer Teilchenquelle 40 verwendet wird, die Teilchen 42a, 42b, 42c abstrahlt. Die Teilchen 42a, 42b, 42c treffen entlang der jeweiligen Strahlrichtung 44a, 44b, 44c auf dem Sensor 10 auf, wobei das Maskenmaterial 22 bzw. die Maske 28 ein Eindringen der Teilchen 44b, 44c in das Substrat 12 verhindern. Das Maskenmaterial 22 und das Material der Maske 28 sind dabei im Zusammenhang mit der jeweiligen Dicke so gewählt, dass die Teilchen 44b, 44c für zu erwartende bzw. gewünschte kinetische Energien sicher aufgehalten wird.

Nur das Teilchen 44a trifft genau die Öffnungen 26, 30 und kann dadurch die erste Schicht 14 durchdringen und in das Substrat 12 eindringen.

Die erste Schicht 14 besteht aus Graphit, dessen elektrischer Widerstand sich in Bezug auf einen ungestörten Zustand gegenüber einem Zustand mit einem Atomgitterdefekt ändert. Genauer gesagt weist die erste Schicht 14 aus Graphit Halbleitereigenschaften auf und besitzt bei Raumtemperatur eine kleine Energielücke von 30 meV und eine sehr geringe Leitungselektronendichte von ≤10¹⁸ cm⁻³. Dadurch kann das Eindringen auch für sehr kleine kinetische Teilchenenergien sicher festgestellt werden.

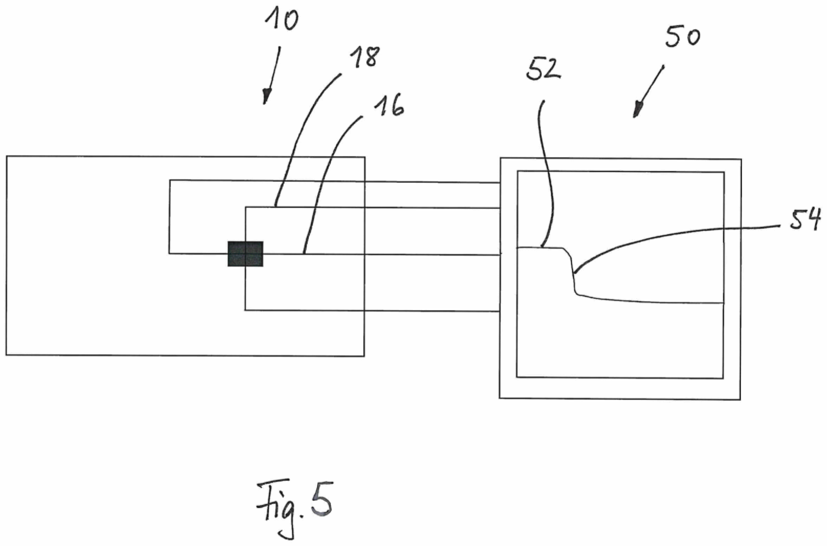

Dieses Feststellen des die erste Schicht 14 durchdringenden Teilchens 42a erfolgt dadurch, dass - wie in Fig. 5 gezeigt ist - der Sensor 10 mit seinen elektrischen Leitungen 16, 18 mit einer Auswerteinheit 50 leitend verbunden ist, die den elektrischen Widerstand 52 im Rahmen einer bekannten Vierpunktmessung in der ersten Schicht 14 bestimmt.

Wenn das Teilchen 42a die erste Schicht 14 durchdringt, werden ein oder mehrere Atomgitterdefekte erzeugt, wodurch der elektrische Widerstand 52 der ersten Schicht 14 schlagartig sinkt 54. Dadurch kann sicher ermittelt werden, dass das Teilchen 42a die erste Schicht durchdrungen hat und somit in das Substrat 12 eingedrungen ist.

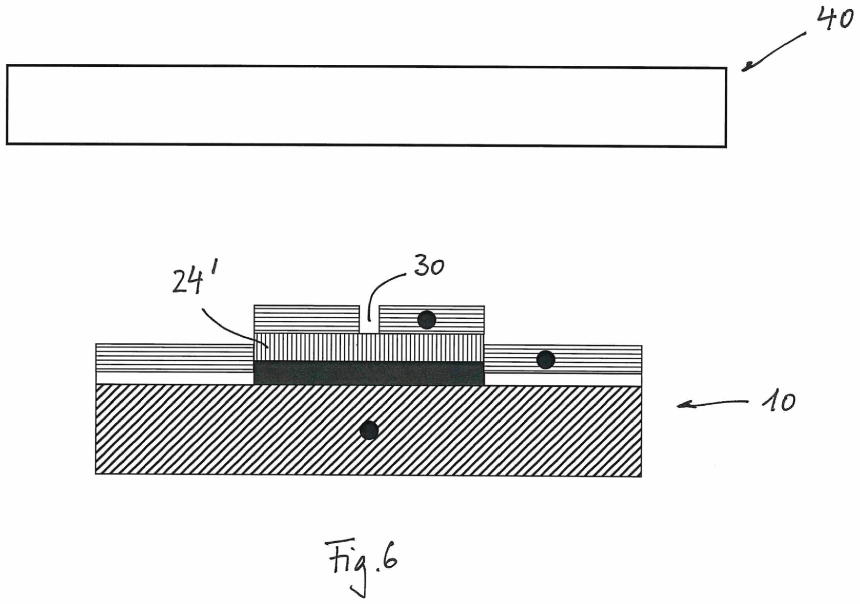

Sobald diese Widerstandsänderung festgestellt wurde, wird über die elektrischen Leitungen 16, 18 der ersten Schicht 14 Energie zugeführt, die als ohmscher Heizer wirkt und sich dadurch aufheizt. Durch dieses Aufheizen auf ca. 100°C wird die zweite Schicht 24 zum Schmelzen gebracht, wodurch eine geschlossene zweite Schicht 24' entsteht, bei der die Öffnung 26 der zweiten Schicht 24 verschlossen ist (vgl. Fig. 6). Zugleich könnte durch eindringendes Schmelzmaterial der zweiten Schicht 24 auch die Öffnung 30 der Maske 28 verschlossen werden (nicht gezeigt).

In jedem Fall wirkt die zweite Schicht 24' nun wie eine Schutzschicht, so dass neu eingestrahlte Teilchen 42d nicht mehr in das Substrat 12 eindringen können. Daher könnte man auch ganz auf die zusätzliche Maske 28 verzichten und nur die zweite Schicht 24 als Maske verwenden.

Die Bestimmung des eingedrungen Teilchens 42a (dauert ca. 1 µs) nach dem tatsächlichen Eindringen) als auch der Verschluss der Öffnung 26 (dauert ca. 5 ms nach dem Feststellen des Eindringens) erfolgen sehr schnell, so dass keine Gefahr besteht, dass in der Zwischenzeit weitere Teilchen 42a in das Substrat 12 eindringen.

Wenn die Heizenergie aus der ersten Schicht 14 nicht ausreicht, könnte man auch zusätzliche Heizmittel (nicht gezeigt) verwenden, die beispielsweise als zusätzliche ohmsche Heizschicht ausgebildet sind.

Andererseits könnte man anstelle einer Aufheizung der zweiten Schicht durch einen ohmschen Heizer auch einen LASER-Strahl oder einen Elektronenstrahl zum Aufschmelzen verwenden.

Weiterhin könnte die Maskenöffnung auch zugewachsen werden. Dazu könnte man einen fokussierter zweiten Ionenstrahl oder einen Elektronenstrahl im Zusammenhang mit einem Precursor-Gas verwenden, um die Öffnungen 26 oder 30 durch Materialabscheidung aus dem Precoursor-Gas zu verschließen nachdem durch den Sensor 10 ein Ionen-Einschlag festgestellt wurde. Hierzu wird der Ionenstrahl zur Ionenimplantation kurzfristig gestoppt und die entsprechende Öffnung 26, 30 verschlossen, bevor die Implantation fortgesetzt wird.

Neben der ortsaufgelösten Elektronen- oder Ionenstrahl gestützten Deposition von Verschlussmaterial aus einem Precursor-Gas kann auch eine Deposition mit einer gelochten AFM Spitze erreicht werden. Eine weitere Methode wäre das mechanische Schließen durch Materialverschiebung mittels einer AFM-Spitze oder dadurch, dass über die AFM-Spitze der Wärmeeintrag zum Aufschmelzen erfolgt (z.B. mit einer Diamantspitze).

Bei einem Substrat mit sehr vielen Maskenöffnungen (z.B. 1000) wäre es vorteilhaft, das Schließen der Öffnungen zu bündeln. Nach einer geeigneten Zeit werden die Widerstandsänderungen mit Hilfe des Sensors 10 bestimmt und diese Öffnungen gleichzeitig verschlossen.

Durch den Sensor 10 kann man somit sicherstellen, dass nur ein Teilchen 42a in das Substrat 12 in einem bestimmten Bereich des Substrates 12, der durch die Öffnungen 26, 30 über der Insel 14 bestimmt wird, eindringen kann.

Mit Hilfe des Sensors 10 kann somit mit einer gewöhnlichen Breitbandteilchenquelle 40 (beispielsweise eine Kaufman-Ionenquelle) eine deterministische Teilchenimplantation mit hoher örtlicher Auflösung auf einfache und kostengünstige Art und Weise erfolgen

Es ist dabei allerdings nicht notwendig, dass die Teilchenquelle geladene Teilchen abstrahlt, es kann sich auch um neutrale Teilchen handeln.

Dies kann beispielsweise für die deterministische Implantation von Stickstoff, Phosphor, Schwefel, Kohlenstoff, Bor oder dergleichen Ionen in Diamant, Silizium, Siliziumkarbid oder dgl. verwendet werden.

Durch die Gitterstruktur 20 ist diese Implantation hochgradig parallel möglich, da sowohl die Detektion des eingedrungenen Teilchens 42a als auch der Verschluss der Öffnungen 28 unabhängig (gleichzeitig oder zeitversetzt) für alle Inseln 14, 14a, 14b, 14c, ..., 14n vorgenommen werden können.

Außerdem kann der Sensor 10 nach der Implantation selbst als Vorrichtung mit zahlreichen Quantensystemen oder zur Herstellung eines Quantencomputers verwendet werden. Dann dienen die elektrischen Leitungen 16, 18 zugleich für die Qubit-Ansteuerung und -Auslese.

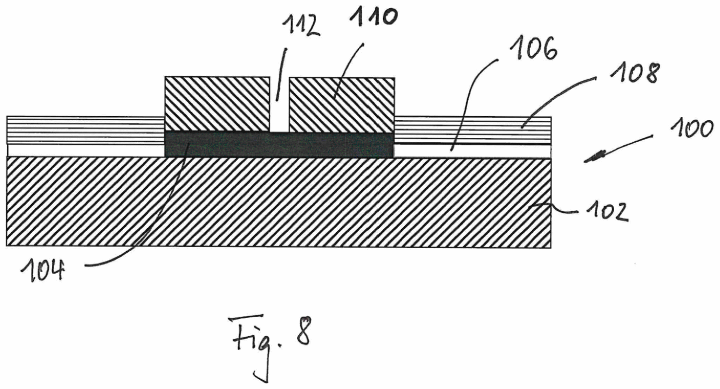

In den Fig. 8 bis 10 ist eine zweite bevorzugte Ausgestaltung des erfindungsgemäßen Sensors 100 in Querschnittsansichten gezeigt.

Es ist in Fig. 8 zu erkennen, dass dieser Sensor 100 ebenfalls ein Substrat 102 aufweist, auf dem eine inselförmige erste Schicht 104 besteht, die mit elektrischen Leitungen 106 elektrisch kontaktiert ist. Auf den elektrischen Leitungen befindet sich wiederum das Maskenmaterial 108.

Im Gegensatz zum Sensor 10 gibt es bei diesem Sensor 100 allerdings keine zweite Schicht, die geschmolzen werden kann, und die Maske 110 mit der zentralen Öffnung 112 über der ersten Schicht 104 ist als Elektrode ausgebildet, besteht also aus einem Metall, das elektrisch kontaktiert ist.

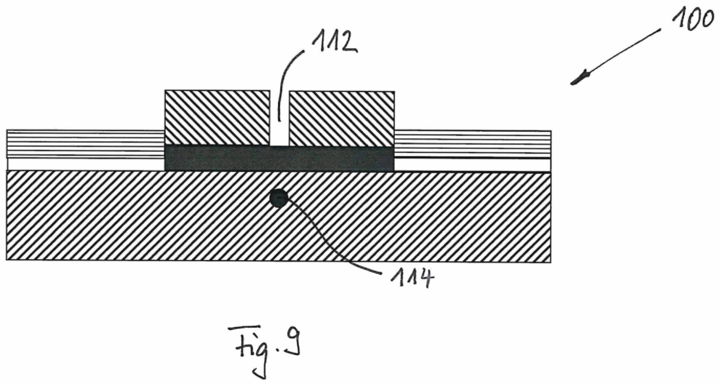

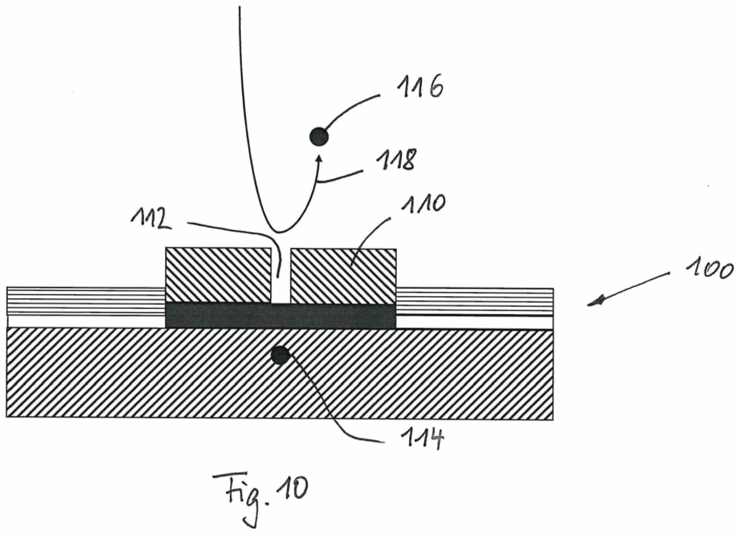

Wenn hier wiederum entsprechend Fig. 5 durch Feststellung einer plötzlichen Widerstandsänderung der ersten Schicht 104 bestimmt wird, dass ein Teilchen 114 durch die Öffnung 112 in das Substrat 102 eingedrungen ist (vgl. Fig. 9), kann das Eindringen weiterer Teilchen 116 nun dadurch unterbunden werden, dass an die Elektrode 110 eine zur Ladung des Teilchens 116 entgegengesetzte Spannung angelegt wird, wodurch das Teilchen 116 von der Elektrode 110 abgestoßen 118 wird (vgl. Fig. 10).

Auch dadurch kann eine deterministische Teilchenimplantation mit hoher örtlicher Auflösung auf einfache und kostengünstige Art und Weise realisiert werden. Dabei ist allerdings notwendig, dass die Teilchenquelle geladene Teilchen abstrahlt, damit diese durch die Elektrode abgelenkt werden können.

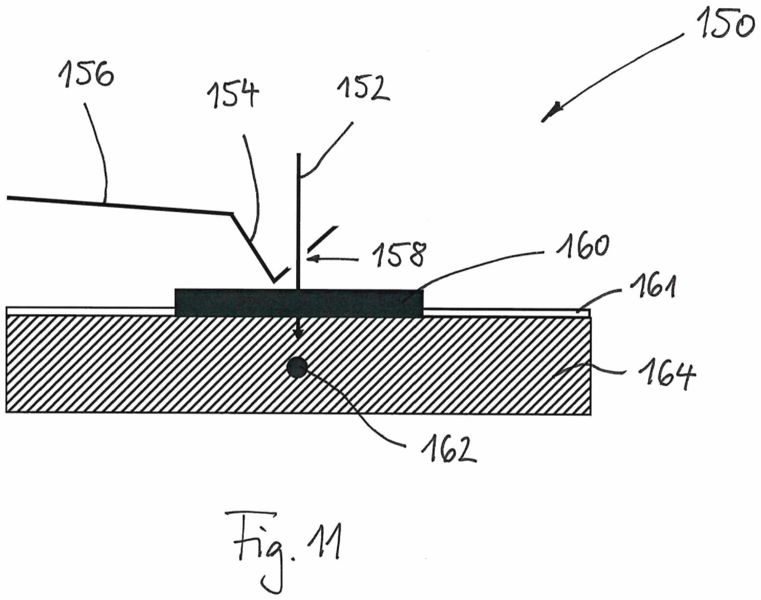

Auf eine Maske 28, 110 und das Maskenmaterial 22, 108 könnte auch ganz verzichtet werden, wenn man entsprechend der in Fig. 11 gezeigten dritten bevorzugten Ausgestaltung des erfindungsgemäßen Sensors 150 den Teilchenstrahl 152 beispielsweise durch die Spitze 154 eines AFM-Cantilevers 156, in der eine Apertur 158 eingebracht ist, steuert, so dass die hohe Ortsauflösung schon im Teilchenstrahl 152 selbst besteht.

Dann muss man nur noch mithilfe der ersten Schicht 160 und den elektrischen Leitungen 162 das eindringende Teilchen 162 bestimmen, um anschließend den Teilchenstrahl 152 abzuschalten und/oder den Teilchenstrahl 152 an einen anderen Ort auf dem Substrat 164 zu verbringen, um dort Teilchen 162 zu implantieren. Auch dann erhält man eine deterministische Teilchenimplantation. Alternativ könnte man aber auch wieder eine Schmelzschicht vorsehen oder eine Elektrode.

Aus der vorstehenden Darstellung ist deutlich geworden, dass mit der vorliegenden Erfindung eine Methode bereitgestellt wird, durch die eine hoch ortsaufgelöste Teilchendetektion, insbesondere eine Einzelteilchendetektion, in einfacher und kostengünstiger Art und Weise bereitgestellt werden kann. Damit kann das Eindringen von Teilchen in ein Material schnell und sicher detektiert werden. Außerdem lässt sich mit der vorliegenden Erfindung eine deterministische Teilchenimplantation in einfacher und kostengünstiger Art und Weise verwirklichen.

Die Erfindung ist ausschließlich durch die beigefügten Ansprüche definiert.

Die Beschreibung dient lediglich der Erläuterung möglicher Ausführungsformen und schränkt den Schutzbereich der Ansprüche nicht ein. Alle in der allgemeinen Beschreibung der Erfindung, der Beschreibung der Ausführungsbeispiele, den nachfolgenden Ansprüchen und in der Zeichnung dargestellten Merkmale können sowohl einzeln als auch in beliebiger Kombination miteinander erfindungswesentlich sein. Diese Merkmale bzw. Merkmalskombinationen können jeweils eine selbständige Erfindung begründen, deren Inanspruchnahme sich ausdrücklich vorbehalten wird. Dabei müssen einzelne Merkmale aus der Beschreibung eines Ausführungsbeispiels nicht zwingend mit ein oder mehreren oder allen anderen in der Beschreibung dieses Ausführungsbeispiels angegebenen Merkmale kombiniert werden, diesbezüglich ist jede Unterkombination ausdrücklich mit offenbart. Außerdem können gegenständliche Merkmale der Vorrichtung umformuliert auch als Verfahrensmerkmale Verwendung finden und Verfahrensmerkmale können umformuliert als gegenständliche Merkmale der Vorrichtung Verwendung finden. Eine solche Umformulierung ist somit automatisch mit offenbart.

### Bezugszeichenliste

- 10: erfindungsgemäßer Sensor gemäß einer ersten bevorzugten Ausgestaltung
- 12: Substrat, Material
- 14, 14a, 14b, 14c, ..., 14n: erste Schicht, Inseln
- 16, 16a, 16b, 16c, ..., 16n,: elektrische Leitungen
- 18, 18a, 18b, 18c, ..., 18n: elektrische Leitungen
- 20, 20a, 20b, 20c, ..., 20n: Gitterstruktur
- 22: Maskenmaterial
- 24: zweite Schicht
- 26: zentrale Öffnung
- 28: Maske
- 30: zentrale Öffnung
- 40: Teilchenquelle
- 42a, 42b, 42c, 42d: Teilchen
- 44a, 44b, 44c: Strahlrichtung
- 50: Auswerteinheit
- 52: elektrischer Widerstand der ersten Schicht 14
- 54: schlagartig sinkender elektrischer Widerstand 52
- 100: erfindungsgemäßer Sensor gemäß einer zweiten bevorzugten Ausgestaltung
- 102: Substrat, Material
- 104: erste Schicht, Insel
- 106: elektrische Leitungen
- 108: Maskenmaterial
- 110: Maske, Elektrode
- 112: zentrale Öffnung in Maske 110
- 114: eingedrungenes Teilchen
- 116: weitere Teilchen
- 118: Abstoßung von Teilchen 116 durch Elektrode 110
- 150: erfindungsgemäßer Sensor gemäß einer dritten bevorzugten Ausgestaltung
- 152: Teilchenstrahl
- 154: Spitze eines AFM-Cantilevers 156
- 156: AFM-Cantilever
- 158: Apertur
- 160: erste Schicht
- 161: elektrische Leitungen
- 162: Teilchen
- 164: Substrat, Material

## Patentansprüche

1. Vorrichtung (10; 100; 150) zur Bestimmung der Implantation eines Einzelteilchens (42a, 42b, 42c, 42d; 114, 116; 162) aus einer Teilchenquelle (40; 152) in ein Material (12; 102; 164), wobei die Vorrichtung (10; 100; 150) die Teilchenquelle (40; 152) und das Material (12; 102; 164) umfasst, **dadurch gekennzeichnet, dass** die Vorrichtung weiterhin eine erste Schicht (14; 104; 160) aufweist, wobei die erste Schicht (14; 104; 160) einen der Stoffe aus der Gruppe: Graphit, Graphen, Bismut, Zinktellurid, Silberselenid und Quecksilbertellurid umfasst und zwischen Teilchenquelle (40; 152) und Material (12; 102; 164) angeordnet ist, wobei die erste Schicht (14; 104; 160) ihren Widerstand ändert, wenn das Einzelteilchen (42a; 114; 162) die erste Schicht (14; 104; 160) durchdringt, wobei die erste Schicht (14; 104; 160) eine Schicht ist, deren elektrischer Widerstand sich in Bezug auf einen ungestörten Zustand gegenüber einem Zustand mit einem Atomgitterdefekt ändert, wobei die Vorrichtung (10; 100; 150) zudem Mittel (16, 18; 106; 161) zur Bestimmung der Widerstandsänderung der ersten Schicht (14; 104; 160) und eine Auswerteeinheit (50), die die elektrische Widerstandsänderung der ersten Schicht (14; 104; 160) überwacht und mit einem Durchdringen der ersten Schicht (14; 104; 160) durch ein Einzelteilchen (42a, 42b, 42c, 42d; 114, 116; 162) korreliert, umfasst.

2. Vorrichtung (10; 100; 150) nach Anspruch 1, **dadurch gekennzeichnet,**
**dass** die erste Schicht (14; 104; 160) sich in ihrer chemischen Zusammensetzung von dem Material (12; 102; 164) unterscheidet und/oder
**dass** die erste Schicht (14; 104; 160) von dem Material (12; 102; 164) beabstandet angeordnet ist, wobei zwischen Material und erste Schicht bevorzugt eine elektrisch isolierende Schicht angeordnet ist, und/oder
**dass** die erste Schicht (14; 104; 160) so ausgebildet ist, dass sich ihr ohmscher Widerstand nach Durchqueren des Teilchens dauerhaft, bevorzugt durch eine Zerstörung ihrer Gitterstruktur ändert und/oder
**dass** die erste Schicht (14; 104; 160) auf dem Material (12; 102; 164) angeordnet ist und/oder
**dass** die erste Schicht (14; 104; 160) als Gitterstruktur ausgebildet ist und/oder
**dass** die erste Schicht (14; 104; 160) eine Schicht (14; 104; 160) ist, die Halbleitereigenschaften aufweist und bei Raumtemperatur eine kleine Energielücke und eine sehr geringe Leitungselektronendichte besitzt, und/oder
**dass** die erste Schicht (14; 104; 160) eine Dicke im Bereich 5 nm bis 100 nm, bevorzugt im Bereich 15 nm bis 25 nm aufweist.

3. Vorrichtung (10; 100; 150) nach Anspruch 1 oder 2, **dadurch gekennzeichnet, dass** die erste Schicht (14; 104; 160) inselförmig ausgebildet ist, wobei die Inseln (14; 104; 160) über elektrische Leitungen (16, 18; 106; 161) verbunden sind, wobei die Inseln (14; 104; 160) bevorzugt eine größere Dimension als die Leitungen (16, 18; 106; 161) und/oder denselben Stoff wie die Inseln aufweisen.

4. Vorrichtung (10; 100; 150) nach Anspruch 3, **dadurch gekennzeichnet,**
**dass** die Inseln (14; 104; 160) eine Dimension im Bereich 1 nm x 1 nm bis 500 nm x 500 nm, vorzugsweise eine Dimension im Bereich 10 nm x 10 nm bis 100 nm x 100 nm, bevorzugt eine Dimension im Bereich 15 nm x 15 nm bis 50 nm x 50 nm, bevorzugt eine Dimension im Bereich 20 nm x 20 nm bis 30 nm x 30 nm aufweisen, und/oder
**dass** die Inseln (14; 104; 160) einen Abstand voneinander im Bereich 10 nm bis 500 nm, bevorzugt im Bereich 20 nm bis 100 nm, insbesondere im Bereich 40 nm bis 60 nm aufweisen.

5. Vorrichtung (100) nach einem der vorherigen Ansprüche, **dadurch gekennzeichnet, dass** zumindest eine Elektrode (110) besteht, die bevorzugt auf der zum Material (102) gegenüberliegenden Seite der ersten Schicht (104) angeordnet ist.

6. Vorrichtung (150) nach einem der vorherigen Ansprüche, **dadurch gekennzeichnet,**
**dass** die Teilchenquelle (152) relativ zum Material (12; 102; 164) bewegbar angeordnet ist und/oder
**dass** die Teilchenquelle (152) eine Apertur (158) aufweist, die an einem AFM-Cantilever (156) besteht.

7. Vorrichtung (10; 100; 150) nach einem der vorherigen Ansprüche, **dadurch gekennzeichnet, dass** auf der zum Material (12; 102; 164) gegenüberliegenden Seite der ersten Schicht (14; 104) zumindest eine Maske (22, 28; 108) angeordnet ist, die für das Teilchen bei gewählten kinetischen Energien undurchdringbar ist, wobei die Maske (22, 28; 108) bevorzugt die erste Schicht (14; 104) zumindest teilweise überdeckt, wobei die Maske (22, 28; 108) insbesondere zumindest eine Öffnung (26; 112) aufweist, die einen Kanal zur Durchleitung des Teilchens (42a; 114) durch die Maske (22, 28; 108) definiert.

8. Vorrichtung (10; 100; 150) nach Anspruch 7, **dadurch gekennzeichnet, dass** die Öffnung (30) der Maske (22, 28; 112) kleiner gleich 100 nm, vorzugsweise kleiner gleich 50 nm, bevorzugt kleiner gleich 10 nm, insbesondere kleiner gleich 5 nm ist.

9. Vorrichtung (10) nach einem der vorherigen Ansprüche, **dadurch gekennzeichnet, dass** auf der zum Material (12) gegenüberliegenden Seite der ersten Schicht (14) zumindest eine durch Aufheizen schmelzbare zweite Schicht (24) angeordnet ist, die für das Teilchen (42d) bei gewählten kinetischen Energien undurchdringbar ist.

10. Vorrichtung (10) nach Anspruch 9, **dadurch gekennzeichnet, dass** ein Heizelement (14) zum Aufheizen der zweiten Schicht (24) besteht, wobei das Heizelement bevorzugt durch die erste Schicht (14) gebildet wird.

11. Vorrichtung (10) nach einem der Ansprüche 7 oder 8 in Kombination mit einem der Ansprüche 9 oder 10, **dadurch gekennzeichnet, dass** die zweite Schicht (24) angrenzend an die Maske (28) besteht, wobei die zweite Schicht (24) bevorzugt zwischen Material (12) und Maske (28) angeordnet ist, wobei insbesondere vorgesehen ist, dass die zweite Schicht (24) im ungeschmolzenen Zustand Durchbrechungen (30) in der Maske (28) nicht überdeckt.

12. Vorrichtung (10; 100; 150) nach einem der Ansprüche 7 bis 11, **dadurch gekennzeichnet,**
**dass** die Maske (22, 28) einen Thermoplast umfasst, bevorzugt PMMA, und/oder
**dass** die zweite Schicht (24) einen der Stoffe umfasst aus der Gruppe: Wachs und Polymer, wobei das Polymer bevorzugt Polycaprolactone (PCL) ist und/oder
**dass** die zweite Schicht (24) einen Schmelzpunkt von kleiner gleich 100 °C aufweist.

13. Verfahren zur Bestimmung der Implantation eines Einzelteilchens (42a, 42b, 42c, 42d; 114, 116; 162) aus einer Teilchenquelle (40; 152) in ein Material (12; 102; 164), **dadurch gekennzeichnet, dass** zwischen Teilchenquelle (40; 152) und Material (12; 102; 164) eine erste Schicht (14; 104; 160) angeordnet wird, die einen der Stoffe aus der Gruppe: Graphit, Graphen, Bismut, Zinktellurid, Silberselenid und Quecksilbertellurid umfasst und die ihren Widerstand ändert, wenn das Teilchen (42a; 114; 162) die erste Schicht (14; 104; 160) durchdringt, wobei die erste Schicht (14; 104; 160) eine Schicht ist, deren elektrischer Widerstand sich in Bezug auf einen ungestörten Zustand gegenüber einem Zustand mit einem Atomgitterdefekt ändert, wobei die Widerstandsänderung der ersten Schicht (14; 104; 160) bestimmt wird und mit einem Durchdringen der ersten Schicht (14; 104; 160) durch ein Einzelteilchen (42a, 42b, 42c, 42d; 114, 116; 162) korreliert wird.

14. Verfahren nach Anspruch 13, **dadurch gekennzeichnet,**
**dass** die Vorrichtung (10; 100; 150) nach einem der Ansprüche 1 bis 12 verwendet wird und/oder
**dass** das Material (12; 102; 164) in Vakuum gehalten wird, wobei der maximale Druck bevorzugt kleiner oder gleich 0,0001 mbar ist, und/oder
**dass** das Material (12; 102; 164) einen der Stoffe umfasst aus der Gruppe: Diamant, SiC, Silizium, TiO₂ und ZnO.

15. Verfahren nach einem der Ansprüche 13 oder 14, **dadurch gekennzeichnet,**
**dass** das Verfahren zur deterministischen Ionenimplantation, bevorzugt zur Erzeugung von Quantensystemen, verwendet wird und/oder
**dass** das Verfahren zur ortsaufgelösten Einzelteilchendetektion verwendet wird und/oder
**dass** das Verfahren für die ortsaufgelöste Massenspektroskopie verwendet wird und/oder
**dass** das Verfahren für die ionenstrahlgestützte Materialforschung verwendet wird und/oder
**dass** das Verfahren für die Erzeugung bestimmter magnetischer Momente in dem Material (12; 102; 164) verwendet wird.

16. Verwendung der Vorrichtung (10; 100; 150) nach einem der Ansprüche 1 bis 12 zur Herstellung eines Detektors für die Massenspektroskopie, eines Detektors für die Einzelteilchenimplantation, eines Informationsspeichers oder eines Bauteils für einen Quantencomputer, bevorzugt einen Prozessor für einen Quantencomputer.

## Claims

1. Device (10; 100; 150) for determining the implantation of a single particle (42a, 42b, 42c, 42d; 114, 116; 162) from a particle source (40; 152) into a material (12; 102; 164), wherein the device (10; 100; 150) comprises the particle source (40; 152) and the material (12; 102; 164),
**characterized in that**
the device further has a first layer (14; 104; 160), wherein the first layer (14; 104; 160) comprises one of the substances from the group: graphite, graphene, bismuth, zinc telluride, silver selenide, and mercury telluride, and is arranged between particle source (40; 152) and material (12; 102; 164), wherein the first layer (14; 104; 160) changes its resistance when the single particle (42a; 114; 162) penetrates the first layer (14; 104; 160), wherein the first layer (14; 104; 160) is a layer whose electrical resistance changes, relative to an undisturbed state, as compared with a state having an atomic lattice defect,
wherein the device (10; 100; 150) moreover comprises means (16, 18; 106; 161) for determining the change in resistance of the first layer (14; 104; 160), and an evaluation unit (50), which monitors the electrical change in resistance of the first layer (14; 104; 160) and correlates it with a penetration of the first layer (14; 104; 160) by a single particle (42a, 42b, 42c, 42d; 114, 116; 162).

2. Device (10; 100; 150) according to claim 1, **characterized**
**in that** the first layer (14; 104; 160) differs in its chemical composition from the material (12; 102; 164), and/or
**in that** the first layer (14; 104; 160) is arranged spaced apart from the material (12; 102; 164), wherein an electrically insulating layer is preferably arranged between the material and the first layer, and/or
**in that** the first layer (14; 104; 160) is configured in such a way that its ohmic resistance changes permanently after the particle has passed through it, preferably by destruction of its lattice structure, and/or
**in that** the first layer (14; 104; 160) is arranged on the material (12; 102; 164), and/or
**in that** the first layer (14; 104; 160) is configured as a grid structure, and/or
**in that** the first layer (14; 104; 160) is a layer (14; 104; 160) that has semiconductor properties and at room temperature has a small energy gap and a very low conduction-electron density, and/or
**in that** the first layer (14; 104; 160) has a thickness in the range from 5 nm to 100 nm, preferably in the range from 15 nm to 25 nm.

3. Device (10; 100; 150) according to claim 1 or 2, **characterized in that** the first layer (14; 104; 160) is configured in island-shaped form, wherein the islands (14; 104; 160) are connected via electrical lines (16, 18; 106; 161), wherein the islands (14; 104; 160) preferably have a larger dimension than the lines (16, 18; 106; 161) and/or consist of the same substance as the islands.

4. Device (10; 100; 150) according to claim 3, **characterized**
**in that** the islands (14; 104; 160) have a dimension in the range from 1 nm × 1 nm to 500 nm × 500 nm, preferably a dimension in the range from 10 nm × 10 nm to 100 nm × 100 nm, more preferably a dimension in the range from 15 nm × 15 nm to 50 nm × 50 nm, more preferably a dimension in the range from 20 nm × 20 nm to 30 nm × 30 nm, and/or
**in that** the islands (14; 104; 160) have a spacing from one another in the range from 10 nm to 500 nm, preferably in the range from 20 nm to 100 nm, in particular in the range from 40 nm to 60 nm.

5. Device (100) according to one of the preceding claims, **characterized in that** at least one electrode (110) is present, which is preferably arranged on the side of the first layer (104) opposite the material (102).

6. Device (150) according to one of the preceding claims, **characterized**
**in that** the particle source (152) is arranged movably relative to the material (12; 102; 164), and/or
**in that** the particle source (152) has an aperture (158) that is present on an AFM cantilever (156).

7. Device (10; 100; 150) according to one of the preceding claims, **characterized in that** on the side of the first layer (14; 104) opposite the material (12; 102; 164), at least one mask (22, 28; 108) is arranged that is impenetrable to the particle at selected kinetic energies, wherein the mask (22, 28; 108) preferably at least partially covers the first layer (14; 104), and wherein the mask (22, 28; 108) in particular has at least one opening (26; 112) that defines a channel for passing the particle (42a; 114) through the mask (22, 28; 108).

8. Device (10; 100; 150) according to claim 7, **characterized in that** the opening (30) of the mask (22, 28; 112) is less than or equal to 100 nm, preferably less than or equal to 50 nm, more preferably less than or equal to 10 nm, in particular less than or equal to 5 nm.

9. Device (10) according to one of the preceding claims, **characterized in that** on the side of the first layer (14) opposite the material (12), at least one second layer (24) is arranged that can be melted by heating and is impenetrable to the particle (42d) at selected kinetic energies.

10. Device (10) according to claim 9, **characterized in that** a heating element (14) for heating the second layer (24) is present, wherein the heating element is preferably formed by the first layer (14).

11. Device (10) according to one of claims 7 or 8 in combination with one of claims 9 or 10, **characterized in that** the second layer (24) is present adjoining the mask (28), wherein the second layer (24) is preferably arranged between the material (12) and the mask (28), and wherein in particular it is provided that the second layer (24), in the unmelted state, does not cover openings (30) in the mask (28).

12. Device (10; 100; 150) according to one of claims 7 to 11, **characterized in that** the mask (22, 28) comprises a thermoplastic, preferably PMMA, and/or **in that** the second layer (24) comprises one of the substances from the group: wax and polymer, wherein the polymer is preferably polycaprolactone (PCL), and/or **in that** the second layer (24) has a melting point of less than or equal to 100 °C.

13. Method for determining the implantation of a single particle (42a, 42b, 42c, 42d; 114, 116; 162) from a particle source (40; 152) into a material (12; 102; 164), **characterized in that**
between particle source (40; 152) and material (12; 102; 164) a first layer (14; 104; 160) is arranged, which comprises one of the substances from the group: graphite, graphene, bismuth, zinc telluride, silver selenide, and mercury telluride, and which changes its resistance when the particle (42a; 114; 162) penetrates the first layer (14; 104; 160), wherein the first layer (14; 104; 160) is a layer whose electrical resistance changes, relative to an undisturbed state, as compared with a state having an atomic lattice defect, wherein the change in resistance of the first layer (14; 104; 160) is determined and is correlated with a penetration of the first layer (14; 104; 160) by a single particle (42a, 42b, 42c, 42d; 114, 116; 162).

14. Method according to claim 13, **characterized**
**in that** the device (10; 100; 150) according to one of claims 1 to 12 is used, and/or
**in that** the material (12; 102; 164) is kept in vacuum, wherein the maximum pressure is preferably less than or equal to 0.0001 mbar, and/or
**in that** the material (12; 102; 164) comprises one of the substances from the group: diamond, SiC, silicon, TiO2, and ZnO.

15. Method according to one of claims 13 or 14, **characterized**
**in that** the method is used for deterministic ion implantation, preferably for producing quantum systems, and/or
**in that** the method is used for spatially resolved single-particle detection, and/or
**in that** the method is used for spatially resolved mass spectrometry, and/or
**in that** the method is used for ion-beam-assisted materials research, and/or
**in that** the method is used for producing certain magnetic moments in the material (12; 102; 164).

16. Use of the device (10; 100; 150) according to one of claims 1 to 12 for producing a detector for mass spectrometry, a detector for single-particle implantation, an information store, or a component for a quantum computer, preferably a processor for a quantum computer.

## Revendications

1. Dispositif (10 ; 100 ; 150) pour déterminer l'implantation d'une particule individuelle (42a, 42b, 42c, 42d ; 114, 116 ; 162) provenant d'une source de particules (40 ; 152) dans un matériau (12 ; 102 ; 164), le dispositif (10 ; 100 ; 150) comprenant la source de particules (40 ; 152) et le matériau (12 ; 102 ; 164), **caractérisé en ce que** le dispositif présente en outre une première couche (14 ; 104 ; 160), la première couche (14 ; 104 ; 160) comprenant l'une des substances du groupe : graphite, graphène, bismuth, tellurure de zinc, séléniure d'argent et tellurure de mercure, et étant disposée entre la source de particules (40 ; 152) et le matériau (12 ; 102 ; 164), la première couche (14 ; 104 ; 160) modifiant sa résistance lorsque la particule individuelle (42a ; 114 ; 162) traverse la première couche (14 ; 104 ; 160), la première couche (14 ; 104 ; 160) étant une couche dont la résistance électrique varie par rapport à un état non perturbé vis-à-vis d'un état présentant un défaut du réseau atomique, le dispositif (10 ; 100 ; 150) comprenant en outre des moyens (16, 18 ; 106 ; 161) pour déterminer la variation de résistance de la première couche (14 ; 104 ; 160) et une unité d'évaluation (50), qui surveille la variation de résistance électrique de la première couche (14 ; 104 ; 160) et la corrèle avec une traversée de la première couche (14 ; 104 ; 160) par une particule individuelle (42a, 42b, 42c, 42d ; 114, 116 ; 162).

2. Dispositif (10 ; 100 ; 150) selon la revendication 1, **caractérisé**
**en ce que** la première couche (14 ; 104 ; 160) se distingue, par sa composition chimique, du matériau (12 ; 102 ; 164) et/ou
**en ce que** la première couche (14 ; 104 ; 160) est disposée à distance du matériau (12 ; 102 ; 164), une couche électriquement isolante étant de préférence disposée entre le matériau et la première couche, et/ou
**en ce que** la première couche (14 ; 104 ; 160) est conçue de telle sorte que sa résistance ohmique varie durablement après la traversée de la particule, de préférence par une destruction de sa structure cristalline, et/ou
**en ce que** la première couche (14 ; 104 ; 160) est disposée sur le matériau (12 ; 102 ; 164), et/ou
**en ce que** la première couche (14 ; 104 ; 160) est réalisée sous forme de structure réticulaire, et/ou
**en ce que** la première couche (14 ; 104 ; 160) est une couche (14 ; 104 ; 160) présentant des propriétés semi-conductrices et possédant, à température ambiante, une faible bande interdite et une très faible densité d'électrons de conduction, et/ou
**en ce que** la première couche (14 ; 104 ; 160) présente une épaisseur dans la plage de 5 nm à 100 nm, de préférence dans la plage de 15 nm à 25 nm.

3. Dispositif (10 ; 100 ; 150) selon la revendication 1 ou 2, **caractérisé en ce que**
la première couche (14 ; 104 ; 160) est réalisée sous forme d'îlots, les îlots (14 ; 104 ; 160) étant reliés par des conducteurs électriques (16, 18 ; 106 ; 161), les îlots (14 ; 104 ; 160) présentant de préférence une dimension supérieure à celle des conducteurs (16, 18 ; 106 ; 161) et/ou présentant la même substance que les îlots.

4. Dispositif (10 ; 100 ; 150) selon la revendication 3, **caractérisé**
**en ce que** les îlots (14 ; 104 ; 160) présentent une dimension dans la plage de 1 nm x 1 nm à 500 nm x 500 nm, de préférence une dimension dans la plage de 10 nm x 10 nm à 100 nm x 100 nm, de manière préférée une dimension dans la plage de 15 nm x 15 nm à 50 nm x 50 nm, de manière préférée une dimension dans la plage de 20 nm x 20 nm à 30 nm x 30 nm, et/ou
**en ce que** les îlots (14 ; 104 ; 160) présentent entre eux une distance dans la plage de 10 nm à 500 nm, de préférence dans la plage de 20 nm à 100 nm, en particulier dans la plage de 40 nm à 60 nm.

5. Dispositif (100) selon l'une quelconque des revendications précédentes, **caractérisé en ce qu'**il existe au moins une électrode (110), qui est de préférence disposée du côté de la première couche (104) opposé au matériau (102).

6. Dispositif (150) selon l'une quelconque des revendications précédentes, **caractérisé**
**en ce que** la source de particules (152) est disposée de manière mobile relativement au matériau (12 ; 102 ; 164) et/ou
**en ce que** la source de particules (152) présente une ouverture (158), qui existe sur un cantilever AFM (156).

7. Dispositif (10 ; 100 ; 150) selon l'une quelconque des revendications précédentes, **caractérisé en ce qu'**au moins un masque (22, 28 ; 108), qui est impénétrable pour la particule à des énergies cinétiques choisies, est disposé du côté de la première couche (14 ; 104) opposé au matériau (12 ; 102 ; 164), le masque (22, 28 ; 108) recouvrant de préférence au moins partiellement la première couche (14 ; 104), le masque (22, 28 ; 108) présentant en particulier au moins une ouverture (26 ; 112), qui définit un canal pour le passage de la particule (42a ; 114) à travers le masque (22, 28 ; 108).

8. Dispositif (10 ; 100 ; 150) selon la revendication 7, **caractérisé en ce que** l'ouverture (30) du masque (22, 28 ; 112) est inférieure ou égale à 100 nm, de préférence inférieure ou égale à 50 nm, de manière préférée inférieure ou égale à 10 nm, en particulier inférieure ou égale à 5 nm.

9. Dispositif (10) selon l'une quelconque des revendications précédentes, **caractérisé en ce qu'**au moins une deuxième couche (24), fusible par chauffage et impénétrable pour la particule (42d) à des énergies cinétiques choisies, est disposée du côté de la première couche (14) opposé au matériau (12).

10. Dispositif (10) selon la revendication 9, **caractérisé en ce qu'**il existe un élément chauffant (14) pour chauffer la deuxième couche (24), l'élément chauffant étant de préférence formé par la première couche (14).

11. Dispositif (10) selon l'une quelconque des revendications 7 ou 8 en combinaison avec l'une quelconque des revendications 9 ou 10, **caractérisé en ce que** la deuxième couche (24) existe de manière adjacente au masque (28), la deuxième couche (24) étant de préférence disposée entre le matériau (12) et le masque (28), étant en particulier prévu que la deuxième couche (24), à l'état non fondu, ne recouvre pas les ajours (30) dans le masque (28).

12. Dispositif (10 ; 100 ; 150) selon l'une quelconque des revendications 7 à 11, **caractérisé**
**en ce que** le masque (22, 28) comprend un thermoplastique, de préférence du PMMA, et/ou
**en ce que** la deuxième couche (24) comprend l'une des substances du groupe : cire et polymère, le polymère étant de préférence de la polycaprolactone (PCL), et/ou
**en ce que** la deuxième couche (24) présente un point de fusion inférieur ou égal à 100 °C.

13. Procédé pour déterminer l'implantation d'une particule individuelle (42a, 42b, 42c, 42d ; 114, 116 ; 162) provenant d'une source de particules (40 ; 152) dans un matériau (12 ; 102 ; 164), **caractérisé en ce qu'**une première couche (14 ; 104 ; 160) est disposée entre la source de particules (40 ; 152) et le matériau (12 ; 102 ; 164), ladite première couche comprenant l'une des substances du groupe : graphite, graphène, bismuth, tellurure de zinc, séléniure d'argent et tellurure de mercure, et modifiant sa résistance lorsque la particule (42a ; 114 ; 162) traverse la première couche (14 ; 104 ; 160), la première couche (14 ; 104 ; 160) étant une couche dont la résistance électrique varie par rapport à un état non perturbé vis-à-vis d'un état présentant un défaut du réseau atomique, la variation de résistance de la première couche (14 ; 104 ; 160) étant déterminée et corrélée avec une traversée de la première couche (14 ; 104 ; 160) par une particule individuelle (42a, 42b, 42c, 42d ; 114, 116 ; 162).

14. Procédé selon la revendication 13, **caractérisé**
**en ce que**
le dispositif (10 ; 100 ; 150) selon l'une quelconque des revendications 1 à 12 est utilisé et/ou
**en ce que** le matériau (12 ; 102 ; 164) est maintenu sous vide, la pression maximale étant de préférence inférieure ou égale à 0,0001 mbar, et/ou
**en ce que** le matériau (12 ; 102 ; 164) comprend l'une des substances du groupe : diamant, SiC, silicium, TiO₂ et ZnO.

15. Procédé selon l'une quelconque des revendications 13 ou 14, **caractérisé en ce que**
le procédé est utilisé pour l'implantation ionique déterministe, de préférence pour la production de systèmes quantiques, et/ou
**en ce que** le procédé est utilisé pour la détection de particules individuelles à résolution spatiale, et/ou
**en ce que** le procédé est utilisé pour la spectroscopie de masse à résolution spatiale, et/ou
**en ce que** le procédé est utilisé pour la recherche sur les matériaux assistée par faisceau d'ions, et/ou
**en ce que** le procédé est utilisé pour la production de moments magnétiques déterminés dans le matériau (12 ; 102 ; 164).

16. Utilisation du dispositif (10 ; 100 ; 150) selon l'une quelconque des revendications 1 à 12 pour la fabrication d'un détecteur pour la spectroscopie de masse, d'un détecteur pour l'implantation de particules individuelles, d'une mémoire d'informations ou d'un composant pour un ordinateur quantique, de préférence d'un processeur pour un ordinateur quantique.
